(19) Europäisches Patentamt
European Patent Office
Office européen des brevets

(11) **EP 3 810 068 B1**

(12) **EUROPEAN PATENT SPECIFICATION**

(45) Date of publication and mention
of the grant of the patent:
**19.02.2025 Bulletin 2025/08**

(21) Application number: **19729318.6**

(22) Date of filing: **13.06.2019**

(51) International Patent Classification (IPC):
*A61K 8/25* (2006.01)     *A61Q 11/00* (2006.01)
*A61K 8/02* (2006.01)     *A61K 8/21* (2006.01)
*C01B 33/193* (2006.01)

(52) Cooperative Patent Classification (CPC):
**A61K 8/25; A61K 8/02; A61K 8/21; A61Q 11/00;
C01B 33/193;** C01P 2006/12; C01P 2006/90

(86) International application number:
**PCT/EP2019/065562**

(87) International publication number:
**WO 2019/243165 (26.12.2019 Gazette 2019/52)**

(54) **SILICA FOR ORAL CARE COMPOSITIONS**

KIESELSÄURE FÜR EINE MUNDPFLEGEZUSAMMENSETZUNG

SILICE POUR COMPOSITIONS DE SOIN BUCCAL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.06.2018 EP 18305772
12.11.2018 EP 18306479**

(43) Date of publication of application:
**28.04.2021 Bulletin 2021/17**

(73) Proprietor: **RHODIA OPERATIONS
69003 Lyon (FR)**

(72) Inventor: **FERAL-MARTIN, Cédric
77184 EMERAINVILLE (FR)**

(74) Representative: **Kraus & Lederer PartGmbB
Thomas-Wimmer-Ring 15
80539 München (DE)**

(56) References cited:
**EP-A1- 0 396 460      WO-A1-95/18066
US-A1- 2014 127 145**

**Description**

**Technical Field**

The present invention relates to a precipitated silica for use in oral care compositions. In particular the invention relates to a precipitated silica having both abrasion properties as well as good compatibility with therapeutic agents such as stannous fluoride.

**Background of invention**

Oral care products such as toothpastes can provide both therapeutic and cosmetic hygiene benefits. Therapeutic benefits include caries prevention which is typically provided by the use of various fluoride salts; gingivitis prevention by the use of antimicrobial agents; or hypersensitivity control. Cosmetic benefits provided by oral products include the control of plaque and calculus formation, removal and prevention of tooth stain, tooth whitening, breath freshening, and overall improvements in mouth feel impression.

One differentiating factor among oral care products, such as toothpaste, is the active ingredient, fluoride. The most commonly used fluoride sources are typically sodium fluoride, sodium monofluorophosphate, and stannous fluoride. Sodium fluoride and sodium monofluorophosphate are effective sources of fluoride ions that remineralize and strengthen weakened enamel thus allowing fighting cavities. By comparison, stannous fluoride not only delivers cavity fighting fluoride, but it also has antibacterial properties, and it provides an anti-sensitivity mechanism of action. Stannous fluoride has both bactericidal and bacteriostatic properties, which fight plaque and treat/prevent gingivitis. Stannous fluoride also deposits a protective mineral barrier over exposed dentinal tubules to help prevent sensitivity pain from triggers such as hot or cold liquids and foods.

Toothpaste compositions commonly contain a silica abrasive for controlled mechanical cleaning, plaque removal and polishing of teeth. Such silica abrasives are known to interact with other co-ingredients of the compositions such as notably fluorides and zinc compounds. Silicas are also not adequately compatible with tin, strontium and other metallic cations. Such incompatibilities have the consequence that these ingredients are no longer available to elicit their beneficial effects.

EP396460A1 discloses precipitated silica having improved compatibility with metallic cations, in particular Zn, as well as with the fluoride ion. The process disclosed in EP396460A1 for the preparation of said precipitated silica comprises two ageing steps performed at different values of pH: a first ageing step performed at a pH between 6 and 8.5, and a second ageing step performed at a pH between 3 and 5. It has surprisingly been found that by eliminating from the process the second ageing step, the one performed at pH between 3 and 5, it is possible to increase the compatibility of the precipitated silica to stannous ions at levels which are desirable in today's oral care applications.

Objective of the present invention is to provide a silica that has improved compatibility with the active ingredients of the toothpaste composition, in particular with stannous fluoride, and that is effective at removing undesirable plaque deposits with optimum abrasion level.

**Summary of invention**

The above-mentioned objective has been met by a precipitated silica which is characterised by:

- a CTAB surface area $S_{CTAB}$ comprised between 5 and 50 $m^2/g$;
- an abrasion depth value Hm between 3.5 and 14.0 $\mu m$; and
- a stannous ion compatibility of at least 40%.

The CTAB surface area $S_{CTAB}$ is a measure of the external specific surface area as determined by measuring the quantity of N hexadecyl-N,N,N-trimethylammonium bromide adsorbed on the silica surface at a given pH.

The CTAB surface area $S_{CTAB}$ is at least 5 $m^2/g$, typically at least 10 $m^2/g$. The CTAB surface area $S_{CTAB}$ may be greater than 15 $m^2/g$.

The CTAB surface area does not exceed 50 $m^2/g$. The CTAB surface area $S_{CTAB}$ may be lower than 45 $m^2/g$, preferably lower than 42 $m^2/g$.

For applications as an abrasive in toothpaste formulations, advantageous ranges of CTAB surface area $S_{CTAB}$ are from 15 to 42 $m^2/g$, preferably from 15 to 40 $m^2/g$.

The BET surface area $S_{BET}$ of the inventive silica is not particularly limited but it is at least 5 $m^2/g$, typically at least 10 $m^2/g$. BET surface area $S_{BET}$ may in certain instances be greater than 12 $m^2/g$, even greater than 16 $m^2/g$. BET surface area $S_{BET}$ is generally at most 60 $m^2/g$. The BET surface area may advantageously be from 8 to 60 $m^2/g$, even from 10 to 55 $m^2/g$, even from 10 to 50 $m^2/g$, preferably from 15 to 50 $m^2/g$.

The precipitated silica of the invention is characterised by a good balance of abrasive properties, that is ability to remove the pellicle deposit of the teeth without damaging the enamel.

The inventive silica is characterised by an abrasion depth value Hm, as determined using the PMMA abrasion test described hereafter, between 3.5 and 14.0 $\mu$m, preferably between 4.0 and 13.5 $\mu$m.

The inventive silica is advantageously characterised by a compatibility with stannous ions, as determined using the stannous ion compatibility method described hereafter, of at least 40%. Stannous ions are generally Sn(II) ions deriving from $SnF_2$.

The stannous ion compatibility is preferably at least 41%, even at least 42%. The stannous ion compatibility may advantageously be as high as 100%, although values of up to 60 are suitable for most applications.

In an advantageous embodiment the inventive silica has a stannous ion compatibility of 40 to 60%.

The inventive silica exhibits in general a high compatibility with respect to cations which are customarily present in toothpaste compositions. Notable non limiting examples of said cations are for instance, calcium, strontium, barium, manganese, indium, nickel, zinc, titanium, zirconium, silver, palladium, ammonium or amino cations. These cations may be in the form of mineral salts, for example chloride, fluoride, nitrate, phosphate, sulfate or in the form of organic salts such as acetates, citrates.

Advantageously, the inventive silica has a compatibility with zinc, as determined using the Zn compatibility method described hereafter, of at least 50%.

The inventive silica is also provided with good compatibility towards the fluoride ion. A wide variety of fluoride ion-yielding materials can be employed as sources of soluble fluoride in toothpaste compositions. Examples of suitable fluoride ion-yielding materials include, for instance, sodium fluoride, (NaF), stannous fluoride ($SnF_2$), potassium fluoride, (KF), potassium stannous fluoride ($SnF_2$-KF), indium fluoride ($InF_3$), zinc fluoride ($ZnF_2$), ammonium fluoride ($NH_4F$), and stannous chlorofluoride (SnClF).

The fluoride ion compatibility of the inventive silica, measured on NaF solutions, is typically greater than 80%, preferably equal to or greater than 90%.

The inventive silica is further characterised by an oil absorption, measured as bis(2-ethylhexyl)adipate (DOA) absorption, which is between 90 and 180 g/100 g, typically between 95 and 140 g/100 g.

Typically the inventive silica is characterised by a pH between 5.0 and 9.0 and preferably between 5.5 and 8.0.

The inventive silica is characterised by a number of OH groups per surface area, expressed as number of $OH/nm^2$, which is equal to or greater than 15, even greater than 20, preferably greater than 30. The number of OH groups per surface area typically does not exceed 60 $OH/nm^2$.

A second object of the present invention is a process for the preparation of a precipitated silica of the first object. Said process comprises the general steps of a precipitation reaction between a silicate and an acid whereby a silica suspension is obtained, followed by the separation and the drying of the suspension.

The process comprises the steps of:

(i) providing an aqueous silicate solution in a vessel, the concentration of silicate, expressed as $SiO_2$, in said vessel being less than 150 g/L and the pH of the aqueous suspension being comprised between 8.0 and 10.0, preferably between 8.5 and 10.0;

(ii) adding an acid to said aqueous silicate suspension to obtain a pH value for the reaction medium between 5.0 and 9.0;

(iii) perform an ageing step for a period from 10 to 120 minutes;

(iv) adding an acid to the reaction medium to lower the pH to a value of less than 5.0, preferably between 3.0 and 5.0 to obtain a silica suspension;

(v) submitting said silica suspension to filtration to provide a filter cake; and optionally drying, wherein no intermediate ageing step is performed between steps (iv) and (v).

The choice of the acid and of the silicate used in the various steps of the process is made in a way well known in the art. The term "silicate" is used herein to refer to one or more than one silicate which can be added during the course of the inventive process. The silicate is typically selected from the group consisting of the alkali metal silicates. The silicate is advantageously selected from the group consisting of sodium and potassium silicate. The silicate may be in any known form, such as metasilicate or disilicate.

In the case where sodium silicate is used, the latter generally has an $SiO_2/Na_2O$ weight ratio of from 2.0 to 4.0, in particular from 2.4 to 3.9, for example from 3.1 to 3.8.

The silicate may have a concentration of from 3.9 wt% to 25.0 wt%, for example from 5.6 wt% to 23.0 wt%, in particular from 5.6 wt% to 20.7 wt%. The silicate concentration is expressed in terms of % by weight of $SiO_2$.

The term "acid" is used herein to refer to one or more than one acid which can be added during the course of the inventive process. Any acid may be used in the process. Use is generally made of a mineral acid, such as sulfuric acid, nitric acid, phosphoric acid or hydrochloric acid, or of an organic acid, such as carboxylic acids, e.g. acetic acid, formic acid or carbonic acid.

The acid may be metered into the reaction medium in diluted or concentrated form. The same acid at different concentrations may be used in different stages of the process. Preferably the acid is sulfuric acid.

In a preferred embodiment of the process sulfuric acid and sodium silicate are used in all of the stages of the process. Preferably, the same sodium silicate, that is sodium silicate having the same concentration expressed as $SiO_2$, is used in all of the stages of the process.

In step (i) of the process an aqueous silicate solution having a pH from 8.0 to 10.0 is provided in the reaction vessel. The starting solution is an aqueous solution, the term "aqueous" indicating that the solvent is water. Preferably, the starting solution has a pH from 8.5 to 10.0.

The silicate concentration of the aqueous silicate solution provided in the reaction vessel in step (i) is less than 150 g/L. The silicate concentration is typically less than 120 g/L, preferably less than 100 g/L, even less than 50 g/L. The silicate concentration is at least 10 g/L, preferably at least 15 g/L. The silicate concentration may conveniently be between 10 and 100 g/L, typically between 10 and 75 g/L, for example between 10 and 50 g/L.

The starting aqueous silicate solution may be obtained in different manners.

In a first embodiment the starting aqueous silicate solution is obtained by adding an acid to a sodium silicate solution so as to obtain a pH value from 8.0 to 10.0.

Alternatively, the starting aqueous silicate solution may be obtained by simultaneously adding an acid and a silicate to water or an initial silicate solution in such a way that the desired pH and initial silicate concentration are achieved.

The addition of an acid in stage (ii) of the process leads to a drop in the pH of the reaction medium. The pH at the end of step (ii) is lower than the pH of the initial silicate solution. Addition of the acid is carried out until a value of the pH of the reaction medium between 5.0 and 9.0, for example between 6.5 and 8.0, is reached.

The temperature of the reaction medium during step (i) and (ii) of the process is typically between 70 and 97°C, typically between 80 and 95°C.

Once the desired pH value has been reached an ageing of the reaction medium is performed at the pH obtained at the end of stage (ii). This ageing step is generally carried out with stirring of the reaction medium. The duration of ageing step (iii) is from 10 to 120 minutes, in particular from 20 to 100 minutes, more preferably from 30 to 100 minutes.

Ageing step (iii) may be performed at the same temperature as steps (i) and (ii) or at a different temperature. Advantageously, step (iii) is performed at a temperature higher than the temperature of steps (i) and (ii), generally at a temperature between 85 and 100°C.

At the end of step (iii) an acid is added to the reaction medium to lower the pH to a value of less than 5.0, preferably between 3.0 and 5.0 and, even more preferably, between 3.5 and 4.0 (step (iv)). A suspension of precipitated silica is obtained at the end of step (iv).

A liquid/solid separation step is subsequently carried out on the suspension of precipitated silica. No intermediate ageing step is performed between steps (iv) and (v)

The separation step normally comprises a filtration, followed, if necessary, by a washing operation. The filtration is performed according to any suitable method, for example by means of a belt filter, a rotary filter, for example a vacuum filter, or, preferably a filter press. The washing is typically carried out with water and/or with an aqueous acidic solution having a pH of between 2.0 and 7.0. Depending on the case, one or more washing steps may be carried out.

The filter cake may optionally be subjected to a liquefaction operation. The term "liquefaction" is intended herein to indicate a process wherein a solid, namely the filter cake, is converted into a fluid-like mass. The expressions "liquefaction step", "liquefaction operation" or "disintegration" are interchangeably intended to denote a process wherein the filter cake is transformed into a flowable suspension, which can then be easily dried. After the liquefaction step the filter cake is in a flowable, fluid-like form and the precipitated silica is again in suspension.

Preferably, in this preparation process, the suspension of precipitated silica obtained after the liquefaction step exhibits, immediately before it is dried, a solids content of at least 25 wt%, in particular of at least 27 wt% The solid content may be up to 50 wt%. Typically the solid content is between 30 and 40 wt%.

In an advantageous embodiment of the invention during the washing and/or during the liquefaction step of the process an aqueous solution containing salts of metallic cations is added to the precipitated silica. Notable non-limiting examples of suitable metallic cations are for instance selected from the group consisitng of the divalent and/or tetravalent cations of Ti, Sn, Zr, Mg, Ca, Sr, Zn, Ba. The cations are preferably selected from the group consisting of the divalent cations of Zn and Sn. Suitable salts for the preparation of the solutions are for instance sulfates. Typically the concentration of the metallic cation in the solution is at least 0.1 wt%, even at least 0.5 wt%. The cation concentration generally does not exceed 2.0 wt%.

The disintegrated filter cake is subsequently dried. The precipitated silica suspension obtained at the end of the liquefaction step is typically dried. Drying may be carried out using any means known in the art. Preferably, drying is carried out by spray drying. For this purpose, any suitable type of spray dryer may be used, especially a turbine spray dryer or a nozzle spray dryer (liquid-pressure or two-fluid nozzle). In general, when the filtration is carried out by means of a filter press, a nozzle spray dryer is used, and when the filtration is carried out by means of a vacuum filter, a turbine spray dryer is used.

When a nozzle spray dryer is used, the precipitated silica is usually in the form of approximately spherical beads.

After drying, a milling step may then be carried out on the recovered product. The precipitated silica that can then be obtained is generally in the form of a powder.

The invention also relates to oral care compositions, preferably toothpaste compositions, containing the inventive precipitated silica.

Examples of suitable oral care compositions are for instance those described in US5578293, US5004597, US5225177.

Typically toothpaste compositions will contain the inventive silica in an amount between 5 and 60% by weight, preferably between 5 and 40% by weight. Toothpaste compositions comprising the inventive silica have a high compatibility to both Sn(II) and fluoride ions as well as a balanced abrasion level.

Should the disclosure of any patents, patent applications, and publications which are incorporated herein by reference conflict with the description of the present application to the extent that it may render a term unclear, the present description shall take precedence.

The invention will be now described in more detail with reference to the following examples whose purpose is merely illustrative and not limitative of the scope of the invention.

ANALYTICAL METHODS

The physicochemical properties of the precipitated silica of the invention were determined using the methods described hereafter.

Determination of CTAB surface area

CTAB surface area ($S_{CTAB}$) values were determined according to an internal method derived from standard NF ISO 5794-1, Appendix G.

Determination of BET surface area

BET surface area $S_{BET}$ was determined according to the Brunauer - Emmett - Teller method as detailed in standard NF ISO 5794-1, Appendix E (June 2010) with the following adjustments: the sample was pre-dried at 200°C $\pm$10°C; the partial pressure used for the measurement $P/P^0$ was between 0.05 and 0.3.

Determination of pH

The pH is measured according to the following method deriving from the standard ISO 787/9 (pH of a 5% suspension in water):

Equipment: calibrated pH meter (accuracy of reading to 1/100th), combined glass electrode, 200 mL beaker, 100 mL measuring cylinder, balance accurate to within about 0.01 g.

Procedure: 5.00 grams of silica are weighed to within about 0.01 gram into the 200 mL beaker. 95 mL of water, measured from the graduated measuring cylinder, are subsequently added to the silica powder. The suspension thus obtained is vigorously stirred (magnetic stirring) for 10 minutes. The pH measurement is then carried out.

**Determination of abrasion depth Hm**

Abrasivity of silica was determined according to an internal method using poly(methyl methacrylate) (PMMA) plates as a substrate. Cast PMMA plates (Altuglas CN, Atoglas, Shore D hardness 60-70) 89 x 20 x 7.5 mm were used as substrate. On each plate a 3 mm wide zone for brushing (Testing area) was defined using adhesive tape and then sumitted to brushing for 10000 cycles using toothbrushes Brosserie Frangaise, held at 15° angle and under a 240 g load, in the presence of slurries of abrasive silica prepared according to ISO11609:2010 protocol. The abrasion depth (Hm, expressed in $\mu$m) at the end of the brushing cycles was measured across a 20 x 10 mm area including the Testing area by optical profilometry (Altimet Altisurf 500) on rinsed plates. The area around the Testing area was used to define the baseline for the optical profilometry determination.

**Determination of Sn(II) ion compatibility**

The compatibility of precipitated silica with Sn (II) ions was determined using a modification of the method disclosed in EP2349488B1.

The method was performed as follows:

(1) a solution in sodium gluconate was prepared containing 0.45 wt % of $SnF_2$ and from 0.6 to 1.0 wt %of sodium gluconate in a solvent composed of a mix of water and sorbitol (the amount of sorbitol in at most 70%). The amount of sorbitol and sodium gluconate were adjusted to prevent precipitation of stannous hydroxide.

(2) 40 g of silica were dispersed in 200 g of the solution obtained in (1). The pH was adjusted to 6.2-6.3 by addition of NaOH 0.1 N. The suspension thus obtained was stirred for 5 hours at 50°C under nitrogen bubbling to avoid any oxidation.

(3) The suspension was filtered through a 45 mm PVDF filter.

(4) The concentration of Sn(II) was measured in the solution prepared in (1) and in the supernatant obtained in (3) using inductively coupled plasma (ICP-OES using a PlasmaQuant PQ9000 Elite instrument).

The Sn(II) compatibility was calculated as the ratio of Sn(II) ions available in the solution obtained at the end of step (3) with respect to the theoretical value according to the following formula:

$$\text{Sn(II) compatibility (\%)} = \frac{\text{Sn(II) in supernatant (3)}}{\text{Sn(II) in solution (1)}} \times 100$$

Determination of fluoride ion compatibility

The amount of fluoride ions in solution was determined using a fluoride ion selective electrode (Perfection or equivalent). The amount of fluoride ions was determined by means of the software LabX. A calibration curve was done by measuring the potential of two standard solutions (190 ppm and 1900 ppm).

The determination of fluoride ion compatibility of the precipitated silica was determined as follows:

(1) an initial solution containing NaF was prepared in a polypropylene vial of 1000 mL by adding 19.3 g ($\pm$ 0.1) of $NaH_2PO_4$, 51.1 g ($\pm$ 0.1) of $Na_2HPO_4$ and 90 mL of a solution of NaF at 40 g/L and then bringing the volume of the solution to 1000 mL with distilled water. The initial solution has a fluoride ion concentration of 1628 ppm and 0.5 M phosphate buffer.

(2) Preparation of the sample solution: 26 g ($\pm$ 0.05) TISAB-F- were added to 10 mL of initial solution prepared in (1).

(3) Testing of the sample of silica: add 7 g ($\pm$ 0.05) of silica were added to 30 g ($\pm$ 0.05) of initial solution prepared in (1) 1and stirred for 1 hour at 60°C. The silica was separated from the solution by centrifugation at 10,000 rpm for 15 minutes. The fluoride ion concentration in the supernatant solution was then determined.

(4) 26 g ($\pm$ 0.05) TISAB-f - were added to 10 mL of supernatant solution and fluoride ion measured at room temperature.

The fluoride ion compatibility was calculated as the ratio of fluoride ions available in the solution after contact with silica (supernatant of step (3) with respect to the theoretical value according to the following formula:

$$\text{F} - \text{compatibility (\%)} = \frac{\text{F} - \text{in supernatant (3)}}{\text{F} - \text{in solution (1)}} \times 100$$

Oil absorption determination

Oil absorption (DOA number) was determined using a method based on ASTM D 2414 for carbon black modified for precipitated silica. 20 g (+/-0,1 g) of precipitated silica are added to the kneading chamber (Brabender Absorptometer "C") with help to the spatula. Bis(2-ethylhexyl) adipate (DOA, CAS [103-23-]) 20 g (+/- 0,1 g) is added dropwise with a dosing rate of 4 mL/min at room temperature into the mixture with continuous mixing (rotation rate of kneader blades 125 rpm). Only very little force is needed for the mixing incorporation process which is followed by using the digital display. Toward the end of the determination, the mixture becomes pasty, and this is indicated by means of a steep rise in the mixing force required. When the display reaches 0.5 Nm, the kneader and the DOA metering are both switched off via an electrical contact. The synchronous motor for DOA input has coupling to a digital counter, and DOA consumption in mL can therefore be read off. The DOA absorption of a silica is determined as the amount of DOA where the torque is at 70% of its maximum value. This value is determined and calculated by the software ABSORPTOMETER. DOA absorption is reported in g/(100 g).

Silanol density determination

The samples were analyzed using ATD-ATG technique on Mettler's LF1100 thermobalance and a Tensor 27 Bruker spectrometer equipped with a gas cell, with the following program: Temperature rise from 25°C to 1100°C at 10°C/min, under air (60 mL/min), in $Al_2O_3$ crucible of 150 $\mu$L. The silanol density is directly related to the loss of mass between 200°C and 800°C. The loss of mass (%) between 200°C and 800°C is identified as $\Delta W\%$ this value.

The silanol ratio (mmol/g) is defined by:

$$T_{SiOH} = \Delta W*2*1000 / (18.015*100) = 1.11*\Delta W$$

Silanol density ($OH/nm^2$) is calculated by :

$$D = T_{SiOH}*Na / 10^{21}*S_{BET} = T_{SiOH}*602.2/ S_{BET}$$

wherein Na : Avogadro's number

**Example 1**

In a 2500 L stainless steel reactor were introduced: 156.6 L of water and 32.8 kg of a sodium silicate solution ($SiO_2/Na_2O$ ratio = 3.43; $SiO_2$ concentration = 19.6 wt%, used in all the steps of the process). Initial pH of the solution was 9.4.

The solution obtained was stirred and heated to reach 95°C. After this first step, a 7.7 wt% sulfuric acid solution was added at a flow rate of 4.2 kg/min to reach a pH of 8.9 and a neutralization ratio of 88%. The same sulfuric acid solution was used in all steps of the process.

The neutralization ratio is defined by the following relation:

$$\text{Neutralization ratio (\%)} = (\text{number of } H^+ \text{ (mol)})/(\text{number of } OH^- \text{ (mol)}).$$

Over a period of 38.3 min, were simultaneously introduced sodium silicate, (flow rate: 10.1 kg/min), water (flow rate: 8.6 kg/min) and sulfuric acid solution to obtain a total neutralization ratio of 90% and a pH value of 8.9.

At the end of the first simultaneous addition, over a period of 21 min, were introduced: sodium silicate (flow rate: 10.1 kg/min) and sulfuric acid to obtain a neutralization ratio of 90% and a pH value 8.9.

The pH of the reaction medium was then brought to a value of 7.0 with sulfuric acid (flow rate: 5.0 kg/min). An ageing step was carried out over a period of 82 min at pH 7.0. After 82 min, the pH of the reaction medium was brought to a value of 4.0 with sulfuric acid (flow rate: 11.9 kg/min). A suspension of precipitated silica was obtained.

The suspension was filtered and washed on a drum filter. The moisture of the cake was more than 45 wt%. The filter cake obtained was disintegrated mechanically and water was added to obtain a $SiO_2$ suspension at 30 wt% of silica. The pH was adjusted with sulfuric acid to reach value less than 5.0.

The product was dried by atomization. The product was obtained in powder form with a moisture less than 5 wt%. The physicochemical properties of the product are reported in Table I.

**Example 2**

In a 2500 L stainless steel reactor were introduced: 158.3 L of water and 32.7 kg of a sodium silicate solution ($SiO_2/Na_2O$ ratio = 3.45; $SiO_2$ concentration = 19.5 wt%, used in all the steps of the process). Initial pH of the solution was 9.4.

The solution obtained was stirred and heated to reach 95°C. After this first step, sulfuric acid solution (at a concentration of 7.7 wt%; the same solution was used in all steps of the process) was added at a flow rate of 4.0 kg/min to reach a pH of 8.1 and a neutralization ratio of 85%.

Over a period of 38.3 min, were simultaneously introduced: sodium silicate, (flow rate: 11.7 kg/min), water (flow rate: 8.6 kg/min) and sulfuric acid solution to obtain a neutralization ratio of 90% and a pH of 8.1.

At the end of the first simultaneous addition, over a period of 21.1 min, were introduced sodium silicate (flow rate: 10.1 kg/min), and sulfuric acid to obtain a neutralization ratio of 90% and a pH of 8.1

The pH of the reaction medium was brought to a value of 7.0 with sulfuric acid (flow rate: 4.9 kg/min). An ageing step was carried out over a period of 77 min at a pH of 7.0. After 77 min, the pH of the reaction medium was brought to a value of 4.0 with sulfuric acid (flow rate: 11.8 kg/min).

A suspension of precipitated silica was obtained. The suspension was filtered and washed on a filter plate. The moisture of the cake was more than 45 wt%. The filter cake obtained was disintegrated mechanically and water was added to obtain a $SiO_2$ suspension at 30 wt% of silica. The pH was adjusted with sulfuric acid to reach a value of less than 5.0.

The product was dried by atomization. The product obtained was in powder form with a moisture of less than 5 wt%. The physicochemical properties of the product are reported in Table I.

**Example 3**

In a 2500 L stainless steel reactor were introduced: 156.6 L of water and 32.76 kg of a sodium silicate solution ($SiO_2$/$Na_2O$ ratio = 3.45; $SiO_2$ concentration = 19.5 wt%, used in all the steps of the process). Initial pH of the solution was 9.4.

The solution obtained was stirred and heated to reach 95°C. After this first step, a 7.8 wt% sulfuric acid solution (the same solution was used in all steps of the process) was added at a flow rate of 4.0 kg/min to reach a pH of 7.3 and a neutralization ratio of 85.8%.

Over a period of 38.9 min, were simultaneously introduced: sodium silicate (flow rate: 9.9 kg/min), water (flow rate: 8.6 kg/min) and sulfuric acid to obtain a total neutralization ratio equal to 89.0% and a pH of 7.3.

At the end of the first simultaneous addition, over a period of 16.4 min, were introduced: sodium silicate (flow rate: 10.2 kg/min) and sulfuric acid to obtain a neutralization ratio of 88.9% and a pH of 7.3.

The pH of the reaction medium was brought to a value of 7.0 with sulfuric acid at a flow rate of 4.9 kg/min. At pH 7.0, an ageing step was carried out over a period of 80 min. After 80 min, the pH of the reaction medium was brought to a value of 4.0 with sulfuric acid at a flow rate of 11.8 kg/min.

A suspension of precipitated silica was obtained. The suspension was filtered and washed on a filter plate. The moisture of the cake was more than 45 wt%.

The filter cake obtained was disintegrated mechanically and water was added to obtain a $SiO_2$ suspension at 30 wt% of silica. Then pH was adjusted with sulfuric acid to reach value less than 5.0.

The product was dried by atomization. The product obtained was in powder form with a moisture of less than 5 wt%. The physicochemical properties of the product are reported in Table I.

**Comparative Example 1**

In a 170 L stainless steel reactor were introduced: 17.8 L of water and 7 kg of a sodium silicate solution ($SiO_2$/$Na_2O$ ratio = 3.44; $SiO_2$ concentration = 12 wt%). The same sodium silicate solution was used in all the steps of the process.

The obtained solution was stirred and heated to reach 90°C. Once the set temperature was reached sulfuric acid (7.7 wt% solution) was added (flow rate: 491 g/min) until the reaction medium reached the pH value of 9.0. The same sulfuric acid solution was used in all the steps of the process.

Simultaneously, over a period of 60 min, were introduced: sodium silicate, at a flowrate of 1429 g/min, and sulfuric acid. The flow rate of the sulfuric acid was regulated so that the pH of the reaction medium was maintained at a value of 9.0.

At the end of the simultaneous addition, the pH of the reaction medium was brought to a value of 7.0 sulfuric acid. Simultaneously, the reaction medium was heated to 95°C. The rest of the process was carried out at this temperature. A first ageing step was carried out at pH 7.0 over a period of 75 min. After 75 min, the pH of the reaction medium was brought to a value of 4.0 with sulfuric acid (flow rate: 680 g/min). At pH 4.0, a second ageing step was carried out over a period of 10 min to obtain a suspension of precipitated silica.

The suspension of precipitated silica was filtered and washed on a filter plate. The moisture of the cake was more than 30 wt%.

The filter cake obtained was disintegrated mechanically and water was added to obtain a $SiO_2$ suspension having 30 wt% of silica content.

The product was dried by spray drying. The product obtained, in powder form, had a moisture content of less than 7 wt%. The physicochemical properties of the product are reported in Table I.

**Comparative Example 2**

In a 10 L stainless steel reactor were introduced: 0.957 L of water and 385 g of a sodium silicate solution ($SiO_2$/$Na_2O$ ratio = 3.43; $SiO_2$ concentration = 12 wt%). The same sodium silicate solution was used in all the steps of the process.

The obtained solution was stirred and heated to reach 90°C. At this temperature, a sulfuric acid (7.7 wt% solution) was added at a flow rate of 22 g/min until the reaction medium reached the pH value of 9.5. The same sulfuric acid solution was used in all the steps of the process.

Simultaneously, over a period of 55 min, were introduced: sodium silicate (flow rate: 85.2 g/min) and sulfuric acid. The flow rate of the sulfuric acid was regulated so that the pH of the reaction medium was maintained at a value of 9.5.

At the end of the simultaneous addition, the pH of the reaction medium was brought to a value of 7.0 with sulfuric acid. Simultaneously, the reaction medium was heated to 95°C. The rest of the process was carried out at this temperature. A first ageing step was performed at pH 7.0 over a period of 50 min. After 50 min, the pH of the reaction medium was brought to a value of 4.0 with sulfuric acid at a flow rate of 45.4 g/min. At pH 4.0, the reaction medium was submitted to a second ageing step over a period of 30 min and a suspension of precipitated silica was obtained.

The suspension was filtered and washed on a filter plate. The moisture of the cake was more than 30 wt%.

The filter cake obtained was disintegrated mechanically and water was added to obtain a $SiO_2$ suspension containing 30 wt% of silica.

The product was dried by spray drying. The product obtained, in powder form, had a moisture content of less than 7 wt%. The physicochemical properties of the product are reported in Table I.

Table I

|  | Example 1 | Example 2 | Example 3 | Comp. Example 1 | Comp. Example 2 |
|---|---|---|---|---|---|
| BET ($m^2$/g) | 35 | 38 | 48 | 64 | 72 |
| CTAB ($m^2$/g) | 31 | 32 | 37 | 60 | 52 |
| Abrasion depth Hm ($\mu$m) | 8.8 | 13.1 | 11.1 | 4.8 | 6.8 |
| DOA (g/100g) | 113 | 105 | 110 | 129 | 122 |
| pH | 5.9 | 6.1 | 6.3 | 7.0 | 7.1 |
| Compatibility Sn(II) (%) | 45 | 48 | 45 | 37 | 29 |
| Compatibility F-(from NaF) (%) | 92 | 94 | 91 | 92 | 92 |
| OH/$nm^2$ | 51 | 51 | 35 | 25 | 27 |

The data in Table 1 show that the inventive silicas have a higher stannous ion compatibility with respect to silicas obtained with a process as disclosed in EP396460A1.

The compatibility of a known precipitated silica for use in toothpaste compositions, Zeodent® 113 (commercially available from Huber) having a CTAB surface area $S_{CTAB}$ of 53 $m^2$/g, to Sn(II) ions was found to be 24%.

The inventive silica thus provides a high availability of both Sn(II) and fluoride ions in compositions comprising stannous fluoride as well as good abrasion levels (good balance between plaque removal and abrasion).

**Claims**

1. A precipitated silica which is **characterised by**:

    - a CTAB surface area $S_{CTAB}$ comprised between 5 and 50 $m^2$/g,
    - an abrasion value Hm between 3.5 and 14.0 $\mu$m; and
    - a stannous ion compatibility of at least 40%, wherein $S_{CTAB}$, Hm and stannous ion compatibility are determined according to the methods disclosed in the description.

2. Precipitated silica according to claim 1 **characterised by** a number of OH per surface area equal to or greater than 15 OH/$nm^2$.

3. A precipitated silica according to anyone of the preceding claims **characterised by**:

    - a CTAB surface area $S_{CTAB}$ comprised between 15 and 50 $m^2$/g;
    - an abrasion value Hm between 4.0 and 14.0 $\mu$m;
    - a stannous ion compatibility of at least 40%, and
    - a number of OH per surface area equal to or greater than 30 OH/$nm^2$.

4. Precipitated silica according to anyone of the preceding claims wherein the stannous ion compatibility is between 40 and 60%.

5. Precipitated silica according to anyone of the preceding claims wherein the CTAB surface area $S_{CTAB}$ is comprised between 15 and 45 m$^2$/g.

6. Precipitated silica according to anyone of the preceding claims wherein the number of OH per surface area is between 30 and 60 OH/nm$^2$.

7. Precipitated silica according to anyone of the preceding claims which has a fluoride ion compatibility of at least 80%, wherein the fluoride ion compatibility is determined by the method disclosed in the description.

8. A process for the preparation of a precipitated silica of anyone of claims 1 to 7 which comprises the steps of:

   (i) providing an aqueous silicate solution in a vessel, the concentration of silicate, expressed as SiO$_2$, in said vessel being less than 150 g/L and the pH of the aqueous suspension being comprised between 8.0 and 10.0, preferably between 8.5 and 10.0;
   (ii) adding an acid to said aqueous silicate suspension to obtain a pH value for the reaction medium between 5.0 and 9.0;
   (iii) perform an ageing step for a period from 10 to 120 minutes;
   (iv) adding an acid to the reaction medium to lower the pH to a value of less than 5.0, preferably between 3.0 and 5.0 to obtain a silica suspension;
   (v) submitting said silica suspension to filtration to provide a filter cake; and optionally drying,

   wherein no intermediate ageing step is performed between steps (iv) and (v).

9. The process of claim 8 wherein the silicate concentration in the aqueous silicate solution in step (i) is between 10 and 50 g/L.

10. The process of claim 8 or 9 wherein the duration of step (iii) is between 30 and 100 minutes.

11. The process of anyone of claims 8 to 10 wherein step (iii) is performed at a temperature higher than the temperature of steps (i) and (ii).

12. The process of anyone of claims 8 to 11 wherein step (iii) is performed at a pH between 5.0 and 9.0.

13. An oral care composition comprising a precipitated silica of anyone of claims 1 to 7.

14. A toothpaste composition comprising the precipitated silica of anyone of claims 1 to 7 and a therapeutically active amount of stannous fluoride.

**Patentansprüche**

1. Fällungskieselsäure, **gekennzeichnet durch**:

   - eine CTAB-Oberfläche $S_{CTAB}$ zwischen 5 und 50 m$^2$/g;
   - einen Abriebwert Hm zwischen 3,5 und 14,0 $\mu$m; und
   - eine Zinn(II)-Ionen-Verträglichkeit von mindestens 40 %, wobei $S_{CTAB}$, Hm und Zinn(II)-Ionen-Verträglichkeit gemäß den in der Beschreibung offenbarten Methoden bestimmt werden.

2. Fällungskieselsäure nach Anspruch 1, **gekennzeichnet durch** eine Zahl von OH-Gruppen pro Oberfläche gleich oder größer als 15 OH/nm$^2$.

3. Fällungskieselsäure nach einem der vorhergehenden Ansprüche, **gekennzeichnet durch**:

   - eine CTAB-Oberfläche $S_{CTAB}$ zwischen 15 und 50 m$^2$/g;
   - einen Abriebwert Hm zwischen 4,0 und 14,0 $\mu$m;
   - eine Zinn(II)-Ionen-Verträglichkeit von mindestens 40 % und
   - eine Zahl von OH-Gruppen pro Oberfläche gleich oder größer als 30 OH/nm$^2$.

4. Fällungskieselsäure nach einem der vorhergehenden Ansprüche, wobei die Zinn(II)-Ionen-Verträglichkeit zwischen 40 und 60 % liegt.

5. Fällungskieselsäure nach einem der vorhergehenden Ansprüche, wobei die CTAB-Oberfläche $S_{CTAB}$ zwischen 15 und 45 m$^2$/g liegt.

6. Fällungskieselsäure nach einem der vorhergehenden Ansprüche, wobei die Zahl von OH-Gruppen pro Oberfläche zwischen 30 und 60 OH/nm$^2$ liegt.

7. Fällungskieselsäure nach einem der vorhergehenden Ansprüche, die eine Fluoridionen-Verträglichkeit von mindestens 80 % aufweist, wobei die Fluoridionen-Verträglichkeit nach der in der Beschreibung offenbarten Methode bestimmt wird.

8. Verfahren zur Herstellung einer Fällungskieselsäure nach einem der Ansprüche 1 bis 7, das die folgenden Schritte umfasst:

(i) Bereitstellen einer wässrigen Silikatlösung in einem Gefäß, wobei die Konzentration an Silikat, ausgedrückt als SiO$_2$, in dem Gefäß weniger als 150 g/l beträgt und der pH-Wert der wässrigen Suspension zwischen 8,0 und 10,0, vorzugsweise zwischen 8,5 und 10,0, liegt;
(ii) Zugeben einer Säure zu der wässrigen Silikatsuspension zum Erhalt eines pH-Werts für das Reaktionsmedium zwischen 5,0 und 9,0;
(iii) Durchführen eines Alterungsschritts über einen Zeitraum von 10 bis 120 Minuten;
(iv) Zugeben einer Säure zu dem Reaktionsmedium zum Senken des pH-Werts auf einen Wert von weniger als 5,0, vorzugsweise zwischen 3,0 und 5,0, zum Erhalt einer Kieselsäuresuspension;
(v) Filtrieren der Kieselsäuresuspension zur Bereitstellung eines Filterkuchens; und gegebenenfalls Trocknen,

wobei zwischen den Schritten (iv) und (v) kein Zwischenalterungsschritt durchgeführt wird.

9. Verfahren nach Anspruch 8, wobei die Silikatkonzentration in der wässrigen Silikatlösung in Schritt (i) zwischen 10 und 50 g/l liegt.

10. Verfahren nach Anspruch 8 oder 9, wobei die Dauer von Schritt (iii) zwischen 30 und 100 Minuten liegt.

11. Verfahren nach einem der Ansprüche 8 bis 10, wobei Schritt (iii) bei einer Temperatur durchgeführt wird, die höher als die Temperatur der Schritte (i) und (ii) ist.

12. Verfahren nach einem der Ansprüche 8 bis 11, wobei Schritt (iii) bei einem pH-Wert zwischen 5,0 und 9,0 durchgeführt wird.

13. Mundpflegezusammensetzung, umfassend eine Fällungskieselsäure nach einem der Ansprüche 1 bis **7.**

14. Zahnpastazusammensetzung, umfassend die Fällungskieselsäure nach einem der Ansprüche 1 bis 7 und eine therapeutisch wirksame Menge an Zinn(II)-fluorid.

**Revendications**

1. Silice précipitée qui est **caractérisée par** :

- une surface CTAB $S_{CTAB}$ comprise entre 5 et 50 m$^2$/g ;
- une valeur d'abrasion Hm comprise entre 3,5 et 14,0 $\mu$m ; et
- une compatibilité avec les ions stanneux d'au moins 40 %, dans laquelle $S_{CTAB}$, Hm et la compatibilité avec les ions stanneux sont déterminées selon les procédés décrits dans la description.

2. Silice précipitée selon la revendication 1, **caractérisée par** un indice d'OH par surface spécifique égal ou supérieur à 15 OH/nm$^2$.

3. Silice précipitée selon l'une quelconque des revendications précédentes, **caractérisée par** :

- une surface CTAB $S_{CTAB}$ comprise entre 15 et 50 m$^2$/g ;
- une valeur d'abrasion Hm comprise entre 4,0 et 14,0 μm ;
- une compatibilité avec les ions stanneux d'au moins 40 %, et
- un indice d'OH par surface égal ou supérieur à 30 OH/nm$^2$.

4. Silice précipitée selon l'une quelconque des revendications précédentes, dans laquelle la compatibilité avec les ions stanneux est comprise entre 40 et 60 %.

5. Silice précipitée selon l'une quelconque des revendications précédentes, dans laquelle la surface CTAB $S_{CTAB}$ est comprise entre 15 et 45 m$^2$/g.

6. Silice précipitée selon l'une quelconque des revendications précédentes, dans laquelle l'indice d'OH par surface est compris entre 30 et 60 OH/nm$^2$.

7. Silice précipitée selon l'une quelconque des revendications précédentes, qui a une compatibilité avec les ions fluorure d'au moins 80 %, dans laquelle la compatibilité avec les ions fluorure est déterminée par le procédé décrit dans la description.

8. Procédé pour la préparation d'une silice précipitée selon l'une quelconque des revendications 1 à 7, qui comprend les étapes de :

   (i) fourniture d'une solution aqueuse de silicate dans un récipient, la concentration en silicate, exprimée en SiO$_2$, dans ledit récipient étant inférieure à 150 g/L et le pH de la suspension aqueuse étant compris entre 8,0 et 10,0, de préférence entre 8,5 et 10,0 ;
   (ii) ajout d'un acide à ladite suspension aqueuse de silicate pour obtenir une valeur de pH du milieu réactionnel comprise entre 5,0 et 9,0 ;
   (iii) réalisation d'une étape de vieillissement pendant une période de 10 à 120 minutes ;
   (iv) ajout d'un acide au milieu réactionnel pour baisser le pH jusqu'à une valeur inférieure à 5,0, de préférence comprise entre 3,0 et 5,0 pour obtenir une suspension de silice ;
   (v) soumission de ladite suspension de silice à une filtration pour obtenir un gâteau de filtration ; et éventuellement séchage,

   dans lequel aucune étape de vieillissement intermédiaire n'est réalisée entre les étapes (iv) et (v).

9. Procédé selon la revendication 8, dans lequel la concentration de silicate dans la solution aqueuse de silicate dans l'étape (i) est comprise entre 10 et 50 g/L.

10. Procédé selon la revendication 8 ou 9, dans lequel la durée de l'étape (iii) est comprise entre 30 et 100 minutes.

11. Procédé selon l'une quelconque des revendications 8 à 10, dans lequel l'étape (iii) est effectuée à une température supérieure à la température des étapes (i) et (ii).

12. Procédé selon l'une quelconque des revendications 8 à 11, dans lequel l'étape (iii) est effectuée à un pH compris entre 5,0 et 9,0.

13. Composition de soins buccaux comprenant une silice précipitée selon l'une quelconque des revendications 1 à 7.

14. Composition de pâte dentifrice comprenant la silice précipitée selon l'une quelconque des revendications 1 à 7 et une quantité thérapeutiquement active de fluorure stanneux.

## REFERENCES CITED IN THE DESCRIPTION

**Patent documents cited in the description**

- EP 396460 A1
- US 5578293 A
- US 5004597 A
- US 5225177 A
- EP 2349488 B1